# DEMANDE DE BREVET EUROPEEN

(11) **EP 1 203 811 A2**
(43) Date de publication de la demande: **08.05.2002**
(21) Numéro de dépôt: 01402819.5
(22) Date de dépôt: 30.10.2001
(51) Int. Cl.: C12N 5/04, C12N 1/00, C12P 1/00, C12P 39/00, C09B 61/00

(54) **Production de metabolites d'intérêt par co-culture de cellules végétales et de cellules non végétales**

(30) Priorité: 03.11.2000 FR 0014144
(71) Demandeur: L'OREAL, 75008 Paris (FR)
(72) Inventeur: Martin, Richard, 37210 Rochecorbon (FR); Belcour-Castro, Béatrice, 37520 La Riche (FR); Hilaire, Pascal, 37210 Vouvray (FR)
(74) Mandataire: Desaix, Anne

(57) **Abrégé**

L'invention porte sur des co-cultures stables in vitro de cellules d'origine végétale et de phythopathogènes, permettant de produire des substances végétales.

L'invention concerne aussi un procédé de co-culture des cellules d'origine végétale et des phytopathogènes en fermenteur pour produire des substances d'intérêt.

## Description

La présente invention porte sur des procédés de culture cellulaire permettant la synthèse de nouveaux composés, basés sur la co-culture de cellules végétales et de cellules ou organismes d'un règne différent.

Les végétaux supérieurs et les champignons, par la richesse de leur patrimoine génétique, sont capables de produire des composés susceptibles de présenter un intérêt dans de nombreuses applications biotechnologiques, notamment dans l'industrie alimentaire, agrochimique, pharmaceutique ou cosmétique. Ils constituent en effet une source potentiellement inépuisable de molécules nouvelles. Lorsqu'une telle substance possédant une activité intéressante est identifiée, la question de sa synthèse en quantité suffisante pour répondre aux besoins existants doit être envisagée.

Deux principaux modes de production sont possibles : la synthèse chimique, souvent de faible coût lorsqu'elle est relativement peu complexe, et l'extraction du composé à partir de la biomasse végétale ou fongique. La culture *in vitro* est une technique employée pour effectuer la synthèse de ces substances : en s'affranchissant des aléas climatiques et des techniques agronomiques, elle permet de produire de façon uniforme et parfois avec de hauts rendements. Elle permet en outre l'activation de certaines voies métaboliques réprimées lors de la croissance normale de la plante. Cependant, la culture *in vitro* lance également des défis technologiques tels que les cultures axéniques en fermenteur sur de longues périodes, avec des temps de génération élevés pour les cellules végétales.

De nombreuses molécules d'intérêt, produites par les cellules végétales et fongiques en culture, sont des dérivés du métabolisme primaire. Ces dérivés ne sont pas directement nécessaires à la croissance de l'organisme, possèdent une grande variété de structures et d'activités biologiques, dues à la diversification des unités de base engagées dans les voies de synthèse. Ils sont spécifiques et varient d'une espèce à l'autre. Leur synthèse ne se fait pas durant tout le cycle de vie mais est liée à l'état de la culture : elle ne débute que lorsque cet état est atteint, généralement pendant la phase stationnaire de la croissance. Elle est contrôlée par un ensemble de gènes régulant le moment et le niveau d'expression. Les mécanismes de contrôle sont intégrés à la physiologie de l'organisme producteur.

La synthèse des métabolites considérés peut induire un antagonisme plus ou moins marqué avec la réalisation du métabolisme primaire correspondant à la division cellulaire. Elle se déroule généralement en deux phases : une étape de production de biomasse puis une étape de fabrication des métabolites dans un milieu spécial où les tissus ne sont plus en croissance.

La culture *in vitro* de cellules et/ou tissus végétaux est une importante source potentielle de métabolites secondaires, mais leur synthèse naturelle est souvent relativement faible : leur expression est réprimée, et il est parfois nécessaire d'ajouter aux cellules des inducteurs spécifiques pour faire exprimer leur patrimoine génétique dormant et optimiser la production.

Des extraits de phytopathogènes inactivés peuvent ainsi jouer le rôle d'éliciteurs et stimuler la production de molécules intéressantes. L'élicitation consiste à induire une augmentation de la synthèse de certains métabolites secondaires par des cellules végétales ; lors d'une infection par un agent pathogène extérieur, certains gènes dont l'activité est faible, sont stimulés, ce qui entraîne un accroissement du métabolisme secondaire et la synthèse de phytoalexines par la plante. Ces phytoalexines sont des substances anti-microbiennes produites et accumulées en réponse à l'agression par l'organisme phytopathogène. Ce sont des composés issus du métabolisme secondaire et ayant généralement un faible poids moléculaire (*Paxton 1981, Whitehead et Threlfall 1992*).

L'ajout d'éliciteurs à des cultures de cellules végétales est de ce fait envisagé pour améliorer la production de molécules d'intérêt. On distingue deux types d'éliciteurs :
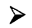 les éliciteurs biotiques : extraits naturels inactivés (par autoclavage ou congélation) comme des broyats de bactéries, de champignons phytopathogènes (hydrates de carbones dérivant de leur paroi cellulaire...)
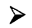 les éliciteurs abiotiques : contraintes et stress dus au froid, aux radiations U.V ...

L'élicitation vise donc à faire produire par les cellules végétales des métabolites secondaires d'intérêt, en grande quantité. Dans le cas où il y a déjà une expression naturelle de ces métabolites, il s'agit d'essayer d'accroître celle-ci en ajoutant des extraits d'éliciteurs biotiques dans le milieu d'alimentation. Dans le cas contraire, il s'agit de stresser les souches pour qu'elles expriment leur patrimoine génétique « dormant » en conditions de confort. Plusieurs méthodes sont envisageables : l'ajout de précurseurs des métabolites considérés, leur couplage avec des éliciteurs, la perméabilisation ou l'immobilisation cellulaire.

Le tableau ci-après présente des exemples de molécules d'intérêt obtenues par élicitation et l'éliciteur utilisé.

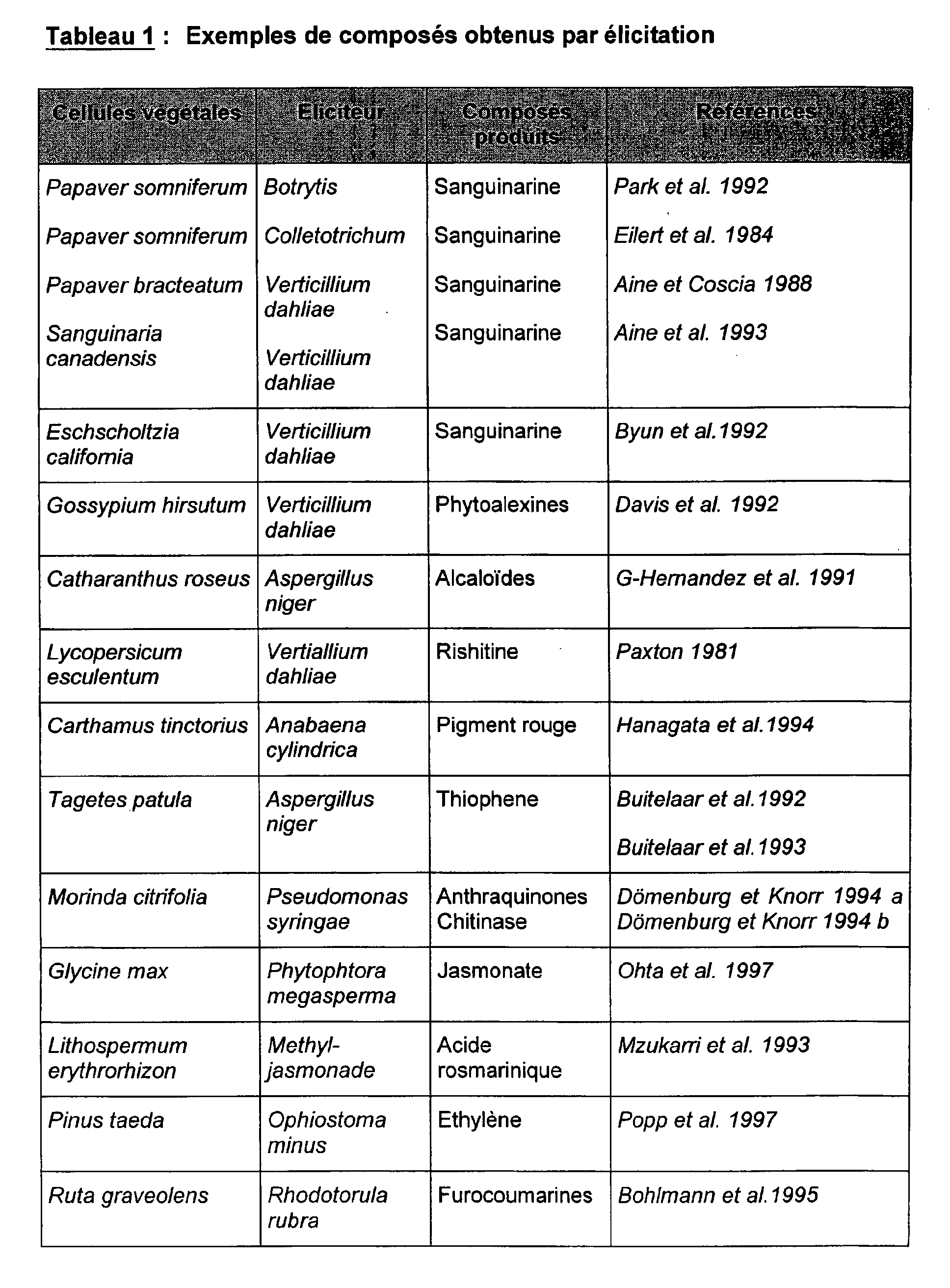

Ainsi, dans des conditions de stress ou d'agression externe, les plantes peuvent activer certaines voies métaboliques et révéler des richesses génétiques réprimées dans des conditions de croissance normales. Par exemple, il est connu que la production de certaines quinones n'est effective que lorsque la plante est agressée par des bactéries ou des fungi. C'est le cas de la plupart des phytopathogènes comme *Verticillium dahliae* (fungus) lorsqu'il parasite le Dahlia ou bien du genre Aspergillus. De nombreux taxons bactériens comme le genre Erwinia produisent les mêmes effets. Les agressions d'insectes (galle du chêne) ou de virus (pigments rouges sur les feuilles de tilleul) provoquent également des réactions de défense capables de générer de nouvelles molécules colorées. La plante réagit en sécrétant des polyphénol oxydases (laccases et catécholases) qui oxydent leurs polyphénols en quinones. Ces dernières polymérisent spontanément au contact de l'oxygène de l'air, créant un film " cicatriciel " bactériostatique et/ou fungistatique, évitant ainsi l'invasivité de l'agresseur exogène.

Chez le Cacao, il y a sécrétion de phytoalexines (acide arjunolique, cyclo-octa-sulfure, phénols). Chez le Coton, il y a induction de la HMGR ( 3-Hydro-3-Méthyl-Glutaryl Co A Réductase), première enzyme intervenant dans le mécanisme primaire de défense et la synthèse du terpène. La plante produit également une phytoalexine sesquiterpénoïde (isoprénoïde) comme facteur de résistance, et du désoxyhémigossypol en réponse à l'infection ; on observe également une peroxidation de la membrane lipidique, la diminution de la concentration en protéines solubles, une variation de la teneur en lipides des racines (baisse de la teneur en lipides totaux, neutres et phospholipides), et la synthèse de pigments phénoliques *(Yunosova et al. 1989, Li et al*. *1995).* Sur l'aubergine, on constate une hausse des activités de la β-1,3-glucanase et de l'amylase dans les feuilles. Chez la pomme de terre, la réponse à l'infection se traduit par une hyper-sensibilité, la synthèse d'une phytoalexine (rishidine), et la subérisation.

Un phénomène analogue est observé chez les phytopathogènes. Lorsqu'un champignon se trouve au contact d'un végétal, son tube germinatif forme un organe spécialisé, l'appressorium, qui sert de base pour la pénétration de la cuticule des cellules végétales. C'est l'action combinée de la pression mécanique et de divers systèmes enzymatiques qui permet cette pénétration. Ainsi, *Verticillium dahliae* produit une protéase alcaline extracellulaire lorsqu'il est en croissance en milieu liquide additionné d'une source protéique. Il possède aussi des enzymes pecto- et protéolytiques : endo-poly-galacturonase, pectine trans-éliminase, pectine méthyl-estérase jouant un rôle pour la pénétration dans l'hôte et la survie du saprophyte, mais pas pour l'infection et l'expression des symptômes de verticillose. Il synthétise de l'éthylène, de l'alcool propylique, de l'éthylacétate, du méthylacétate (phytotoxine).

L'interaction entre un végétal et un micro-organisme entraîne donc souvent une modification du métabolisme d'un des deux organismes, voire des deux organismes à la fois. Par ailleurs, une symbiose peut résulter de l'interaction entre une plante et certaines bactéries ou certains champignons. De tels processus naturels sont déjà avantageusement exploités pour améliorer la croissance de certaines plantes. Ainsi, plusieurs exemples d'interaction entre des végétaux et des bactéries ont été décrits.

Par exemple, des rhizobactéries favorisent la croissance des plantes et les protègent contre les micro-organismes pathogènes. Ainsi, *Pseudomonas fluorescens* M.3.1 stimule la croissance du maïs et limite l'effet néfaste de *Fusadum roseum (Lugtenberg et al. 1991, Benizri et al. 1997).*

Un autre effet d'une bactérie favorisant la croissance des plantes a été étudié : *Pseudomonas sp.* Souche PsJN permet d'augmenter la résistance des jeunes plants de tomate à la flétrissure de *Verticillium dahliae (Sharma et Novak 1998).* Des plantules de tomates d'un cultivar sensible à *Verticillium dahliae* co-cultivées *in vitro* avec la bactérie ont été infectées par *V. dahliae* et des semis colonisés *in vivo* après 3 semaines de croissance en serre. En culture *in vitro,* des différences significatives ont été notées entre les plants co-cultivés avec *Pseudomonas* et les plants témoins, le degré de protection conféré par la colonisation bactérienne étant fonction de la densité de l'inoculum de *V. dahliae*. En culture *in vivo,* c'est seulement après 3 semaines d'exposition au pathogène que des différences de croissance apparaissent. Cela laisse suggérer qu'une colonisation endophyte des tissus de la tomate par la bactérie est nécessaire à l'expression d'une résistance à *V. dahliae* (*Sharma et Nowak 1998).*

D'autres exemples de « biotisation » *in vitro* entre des tissus végétaux et des micro-organismes montrant des effets bénéfiques sont décrits dans un article de revue récent (*Nowak 1998*) : ainsi, une co-culture entre des cotylédons de soja et deux souches de *Pseudomonas maltophilia* stimule le développement de racines de *Brassica campestris* dans des conditions de croissance gnotobiotique.

Les inventeurs ont envisagé la possibilité d'exploiter les modifications métaboliques résultant de l'interaction entre un végétal et un micro-organisme, notamment un phytopathogène, pour produire certaines substances d'intérêt, dans un contexte favorable à une exploitation industrielle.

Il est en effet impossible d'envisager la culture en plein champ de plantes qui seraient par la suite infectées volontairement par des phytopathogènes. En effet, d'une part, les phytopathogènes sont des organismes généralement peu sélectifs et infecteraient rapidement d'autres cultures que celles ciblées, et d'autre part, la zone agricole serait de fait interdite à la production des mêmes genres végétaux.

Les inventeurs ont donc recherché des conditions de production de substances d'intérêt, compatibles avec un milieu de culture confiné. Les exemples de cultures mixtes associant deux partenaires vivants dans un volume confiné, en vue de recueillir les produits de leur métabolisme d'interaction, sont encore très peu nombreux et impliquent des partenaires du même règne biologique.

William *et al.* décrivent par exemple des co-cultures entre bactéries permettant d'augmenter l'utilisation du xylane par *Ruminococcus flavefaciens FDI.* La culture de cette bactérie en association avec *Methanobrevibacter smithii* augmente les activités spécifiques d'enzymes dégradant les polysaccharides extracellulaires. Des interactions sur les transferts d'hydrogène se produisent, la fermentation devient acétogénique, du méthane est formé en lieu et place de l'hydrogène, permettant l'accumulation de xylobiose et de xylose. Des effets similaires sont obtenus par co-culture entre *Ruminococcus flavefaciens* et *Acetitomaculum ruminis (Williams et al. 1994)*.

Mahagamasekera *et al.* décrivent une coculture intergénérique entre des racines transformées par *Agrobacterium rhizogenes* et *Atropa belladonna* et des tiges hybrides de *Duboisia.* Cette co-culture permet la production à un niveau élevé de scopolamine, alors que cette substance est absente des cultures seules. En effet, les plantes produisant de la scopolamine synthétisent de l'hyocyamine au niveau des racines, ce précurseur étant ensuite converti en scopolamine par une enzyme active principalement dans les feuilles. La co-culture de ces deux types de cellules végétales permet donc de rétablir la voie métabolique, avec synthèse d'hyocyamine par les racines et conversion de l'hyocyamine en scopolamine par les tiges *(Mahagamasekera et al*. *1998).*

La présente invention propose d'effectuer des co-cultures de tissus ou cellules d'origine végétale et de tissus ou cellules d'origine non végétale, tels que des phytopathogènes vivants, en fermenteur, afin de produire des substances d'intérêt. Les conditions de confinement permettent de contourner les difficultés de « biosécurité » évoquées plus haut. La co-culture en fermenteur présente par ailleurs d'autres avantages, qui seront présentés plus loin.

Des exemples de phytopathogènes naturels, utilisables dans le cadre de l'invention, sont présentés dans le tableau 2 ci-dessous, en rapport avec des familles botaniques (hôtes) connues pour être ciblées par ces derniers.

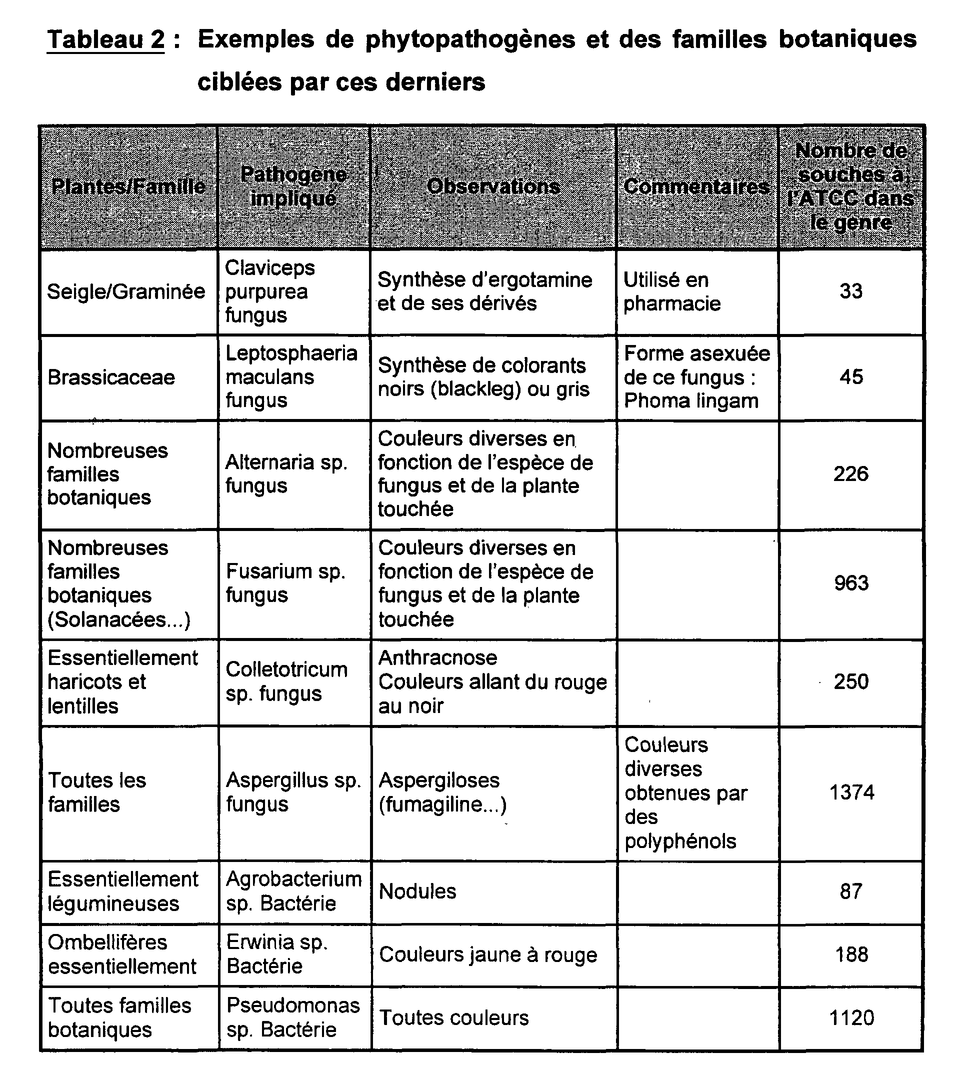

Ces couples phytopathogène/hôte peuvent être utilisés dans les co-cultures de l'invention.

Toutefois, les co-cultures de la présente invention ne sont pas limitées à des co-cultures de cellules d'origine végétale en présence d'un phytopathogène naturel ou avéré du végétal considéré. Par exemple, un pathogène d'une famille botanique donnée peut être co-cultivé avec des cellules provenant d'une famille botanique différente. On peut aussi envisager la co-culture de cellules d'origine végétale avec des cellules ou organismes ne correspondant pas à un phytopathogène identifié comme tel.

En effet, bien que les seules interactions cellules végétaleslphytopathogènes caractérisées à ce jour soient liées à des infections observables dans la nature, l'invention propose de réaliser également des co-cultures de cellules végétales et de cellules avec lesquelles elles n'ont jamais été mises en contact, dans le but d'induire ou de favoriser la synthèse de nouveaux composés. Cette synthèse peut dans ces conditions être le résultat d'une dé-répression de certaines voies métaboliques dormantes chez l'un ou plusieurs des types cellulaires co-cultivés ou de tout autre processus métabolique.

Dans la suite de ce texte, le terme « phytopathogène » désignera donc tout organisme ou cellules autres que des cellules issues de végétaux supérieurs, par exemple des microorganismes tels que les bactéries, archébactéries, cyanobactéries, les virus, les protistes, les levures, les champignons, qu'il s'agisse ou non d'un organisme capable d'infecter naturellement une plante, ou des cellules isolées d'organismes pluricellulaires, notamment des cellules animales, dès lors que l'association *in vitro,* établie dans les conditions de l'invention, entre des cellules végétales et le phytopathogène, conduit à la production de substances d'intérêt.

Le terme « phytopathogène authentique » désignera, dans le cadre de la définition donnée ci-dessus des phytopathogènes, un phytopathogène identifié comme tel au sens littéral dans l'art antérieur, c'est-à-dire un organisme, notamment microorganisme ou virus connu pour induire une maladie chez une plante. Dans cette définition, le terme « maladie » désigne toute inhibition de la croissance, du développement, nécrose des tissus, ou diminution de la fertilité chez une plante. Un phytopathogène sera dit « authentique » quelle que soit l'étendue de son spectre d'hôte naturel, dès lors que ce spectre comporte au moins une espèce végétale. Un phytopathogène authentique peut, selon l'invention, être co-cultivé avec des cellules végétales d'une espèce distincte de son ou ses hôte(s) naturel(s). Ainsi, le Verticillium dahliae est un phytopathogène authentique puisqu'il est connu pour parasiter naturellement un grand nombre d'espèces végétales dont le dahlia, le coton, la pomme de terre, le cacao, la tomate, l'aubergine ou encore le fraisier. Dans l'exemple 2, ce phytopathogène authentique est co-cultivé avec des cellules de Ruta graveolens, qui n'est pourtant pas son hôte naturel privilégié.

Le cas échéant, les co-cultures de l'invention peuvent comporter des cellules végétales de plusieurs types et/ou plusieurs phytopathogènes différents. Dans tous les cas, une co-culture suivant la présente invention comportera au moins un type de cellules d'origine végétale et un type de cellules d'un règne différent, ces dernières étant désignées par le terme « phytopathogène ».

Les co-cultures de la présente invention sont des co-cultures « vraies », ce qui signifie que les différentes cellules et/ou organismes co-cultivés sont vivants lorsqu'ils sont apportés à la culture, par opposition à l'élicitation par mise en contact de cellules végétales avec des extraits naturels inactivés.

Les co-cultures de l'invention se démarquent des cultures dans lesquelles les cellules vivantes sont soit uniquement des cellules végétales, soit uniquement des phytopathogènes, que l'on peut encore appeler « cultures pures ». Un exemple de culture pure de phytopathogène est la culture de *Verticillium dahliae* décrite dans l'exemple 2. Un autre exemple de culture pure est celui d'une culture de cellules végétales en présence d'un éliciteur tel que des bactéries autoclavées.

Selon un mode de réalisation particulier de l'invention, la co-culture est stable, ce qui signifie que les différents types cellulaires en présence sont capables de se reproduire ou à tout le moins de subsister en co-culture, de telle façon qu'un équilibre puisse s'instaurer entre les différentes lignées (végétales et de phytopathogènes) se traduisant par le fait qu'un grand nombre de repiquages successifs de la co-culture n'aboutisse pas à l'élimination d'une des lignées. En pratique, une co-culture peut être considérée comme stable dès lors que tous les types cellulaires en présence sont vivants à l'issue d'un cycle complet de croissance de la cellule végétale autorisant le doublement de la population cellulaire de référence (lorsque plusieurs types de cellules végétales sont présents dans la co-culture, le cycle de croissance considéré sera celui de la cellule la plus lente). En effet, l'expérience montre que si tous les types cellulaires d'une co-culture comportant des cellules végétales et des phytopathogènes sont vivants à l'issue d'un cycle cellulaire du type cellulaire le plus lent, alors les différents types cellulaires seront capables de coexister et un équilibre s'installera entre eux. A l'inverse, si deux types cellulaires ne peuvent pas coexister au sein d'une même culture, ceci se traduira par la mort rapide (quelques jours au maximum) de l'un d'entre eux.

Dans ces co-cultures, les cellules d'origine végétale peuvent être individualisées ou organisées en structures pluricellulaires. En particulier, il peut s'agir de cellules dédifférenciées (exemple 2), ou d'organes, par exemple de racines (exemple1).

Par cellules végétales dédifférenciées, on entend toute cellule végétale ne présentant aucun des caractères d'une spécialisation particulière et capable de vivre par elle-même et non en dépendance avec d'autres cellules. Ces cellules végétales dédifférenciées sont éventuellement aptes, sous l'effet d'une induction, à toute différenciation conforme à leur génome.

Selon la méthode de culture choisie, et en particulier selon le milieu de culture choisi, il est possible d'obtenir à partir d'un même explant des cellules végétales dédifférenciées présentant des caractères différents (Plant propagation by tissue culture, George E. F. and Sherrigton P. D., 1984, Exegetics Limited).

Dans une réalisation préférée des co-cultures de l'invention, les phytopathogènes sont des cellules procaryotes. En particulier, il peut s'agir de bactéries infectant naturellement les plantes dont sont issues les cellules végétales avec lesquelles elles sont co-cultivées. Toutefois, les co-cultures de la présente invention ne sont pas limitées à des co-cultures de cellules d'origine végétale en présence d'un phytopathogène naturel du végétal considéré. Par exemple, un pathogène d'une famille botanique donnée peut être co-cultivé avec des cellules provenant d'une famille botanique différente. On peut aussi envisager la co-culture de cellules d'origine végétale avec des cellules procaryotes ne correspondant pas à un phytopathogène authentique. Une co-culture de cellules végétales et de cellules avec lesquelles elles n'ont jamais été mises en contact est en effet susceptible de modifier certaines voies métaboliques dormantes chez l'un ou plusieurs des types cellulaires co-cultivés, et de permettre ainsi la synthèse de nouveaux composés. Des co-cultures de cellules d'origine végétale avec des bactéries infectant naturellement des animaux, ou des co-cultures de cellules d'origine végétale avec des archébactéries, permettant de produire des substances d'intérêt, font donc également partie de la présente invention.

Dans une autre réalisation préférée des co-cultures de l'invention, les phytopathogènes sont des fungi. En particulier, il peut s'agir de levures ou de champignons inférieurs.

Comme il a été mentionné plus haut, les co-cultures de la présente invention ne sont pas limitées à des co-cultures de cellules végétales avec un phytopathogène authentique. Toutefois, ce type de co-cultures impliquant, d'une part, des cellules végétales (dédifférenciées ou non, le cas échéant organisées en structures pluricellulaires) et, d'autre part, un phytopathogène authentique (dont l'hôte végétal naturel peut être distinct ou non des cellules végétales particulières de la co-culture), constitue un aspect particulier de l'invention.

Selon un autre mode de réalisation particulier de l'invention, la co-culture est une co-culture stable de cellules dédifférenciées et d'un phytopathogène quelconque, en particulier un phytopathogène qui n'a pas pour hôte naturel, les cellules végétales particulières de la co-culture et, le cas échéant, un phytopathogène non authentique, c'est-à-dire n'infectant pas naturellement les plantes.

Dans une autre réalisation des co-cultures de l'invention, les phytopathogènes sont des cellules eucaryotes d'origine animale. Il peut s'agir de cellules primaires ou de cellules d'une lignée cellulaire immortalisée. En outre, ces cellules peuvent être génétiquement modifiées, éventuellement pour sécréter un composé qui interagira avec les cellules végétales ou leurs métabolites. Les cellules animales peuvent être dédifférenciées ou avoir des caractéristiques d'un type cellulaire particulier. Ces cellules peuvent être par exemple issues de mammifères ou d'insectes. Le cas échéant, il peut s'agir d'insectes, à l'état larvaire ou non.

Dans une réalisation particulière des co-cultures de l'invention, les phytopathogènes sont des virus. Ces virus peuvent être des virus infectant naturellement des plantes, comme par exemple, parmi les virus à ADN, ceux de la famille des Caulimovirus comme par exemple le virus de la mosaïque du choux-fleur ou encore, parmi les virus à ARN, ceux de la famille des Bromoviridae comme par exemple le virus de la mosaïque de la luzerne, sans exclure des virus d'un tropisme naturel différent.

Le nombre de types de cellules d'origine végétale présents dans les co-cultures de l'invention n'est pas limité, pas plus que le nombre de types de phytopathogènes co-cultivés avec les cellules végétales. Par exemple, une co-culture de l'invention peut comporter un type donné de cellules végétales, des cellules d'une lignée d'origine animale, et un virus infectant naturellement lesdites cellules d'origine animale. Un autre exemple de culture complexe suivant l'invention comporterait des cellules végétales dérivées de deux taxons botaniques différents, et un phytopathogène.

Dans une réalisation particulière des co-cultures définies précédemment de l'invention, le milieu de culture est avantageusement initialement parfaitement défini. En particulier, le milieu de culture utilisé peut être un milieu entièrement synthétique. Il peut être choisi par exemple parmi les milieux de culture classiquement utilisés pour cultiver des cellules végétales, mais il peut aussi être modifié pour permettre une croissance optimisée des différents types cellulaires co-cultivés.

De façon intéressante, les inventeurs ont observé que des phytopathogènes peuvent participer à l'établissement d'une co-culture stable avec des cellules végétales, en présence d'un milieu de culture qui ne leur est pas spécifique, et ce même dans des conditions de culture (immersion dans le milieu) particulières.

Une co-culture particulière de l'invention est une culture de racines *d'Impatiens balsamina* en présence de *Streptococcus sp.* Une autre co-culture particulière de l'invention est une culture de *Ruta graveolens* dédifférenciées en présence de *Verticillium dahliae*.

Les co-cultures selon l'invention sont réalisées de préférence dans un fermenteur qui peut être n'importe quel type d'enceinte. Cette enceinte est avantageusement stérile et/ou stérilisable, et/ou agitée et/ou soumise à agitation.

Les substances d'intérêt produites par les procédés de l'invention peuvent être destinées à plusieurs types d'applications industrielles, notamment dans les domaines de l'alimentation, l'agrochimie, la pharmacie et la cosmétique. Des co-cultures préférées de l'invention sont donc des co-cultures permettant la production de substances d'intérêt pour l'alimentation, l'agrochimie, la pharmacie ou la cosmétique.

Dans une réalisation particulière de co-culture suivant l'invention, une substance d'intérêt produite par ladite co-culture est synthétisée de façon plus efficace que dans les cultures pures dans le contexte de l'invention. L'initiation ou l'augmentation de la synthèse de la substance d'intérêt est mesurée par rapport à la culture pure qui synthétise ladite substance de la façon la plus efficace. La substance d'intérêt peut être relarguée dans le milieu de culture ou non. Dans ce dernier cas, une étape de lyse des cellules peut être nécessaire pour mesurer la production de ladite substance. Dans une réalisation préférée de co-culture suivant l'invention, le facteur d'augmentation de la synthèse d'une substance d'intérêt est compris entre 2 et 3, ou entre 3 et 10 ; dans certains cas, il peut être supérieur à 10, voire supérieur à 100.

Un type particulier de substances que l'on peut obtenir par les co-cultures de l'invention sont les composés à pouvoir colorant, utilisables notamment en cosmétique. Parmi ces composés, on peut distinguer les colorants et les pigments. Les colorants sont solubles dans un solvant et sont des molécules de taille réduite qui pénètrent facilement le cheveu. Les pigments sont insolubles dans leur milieu d'utilisation. Leur structure est cristalline ou amorphe.

Les premiers composés à pouvoir colorant utilisés étaient d'origine végétale (indigo, garance, campêche), animale (cochenille, pourpre), ou minérale (outremer).

Le tableau 3 ci-après présente plusieurs composés à pouvoir colorant, extraits des végétaux et des champignons.

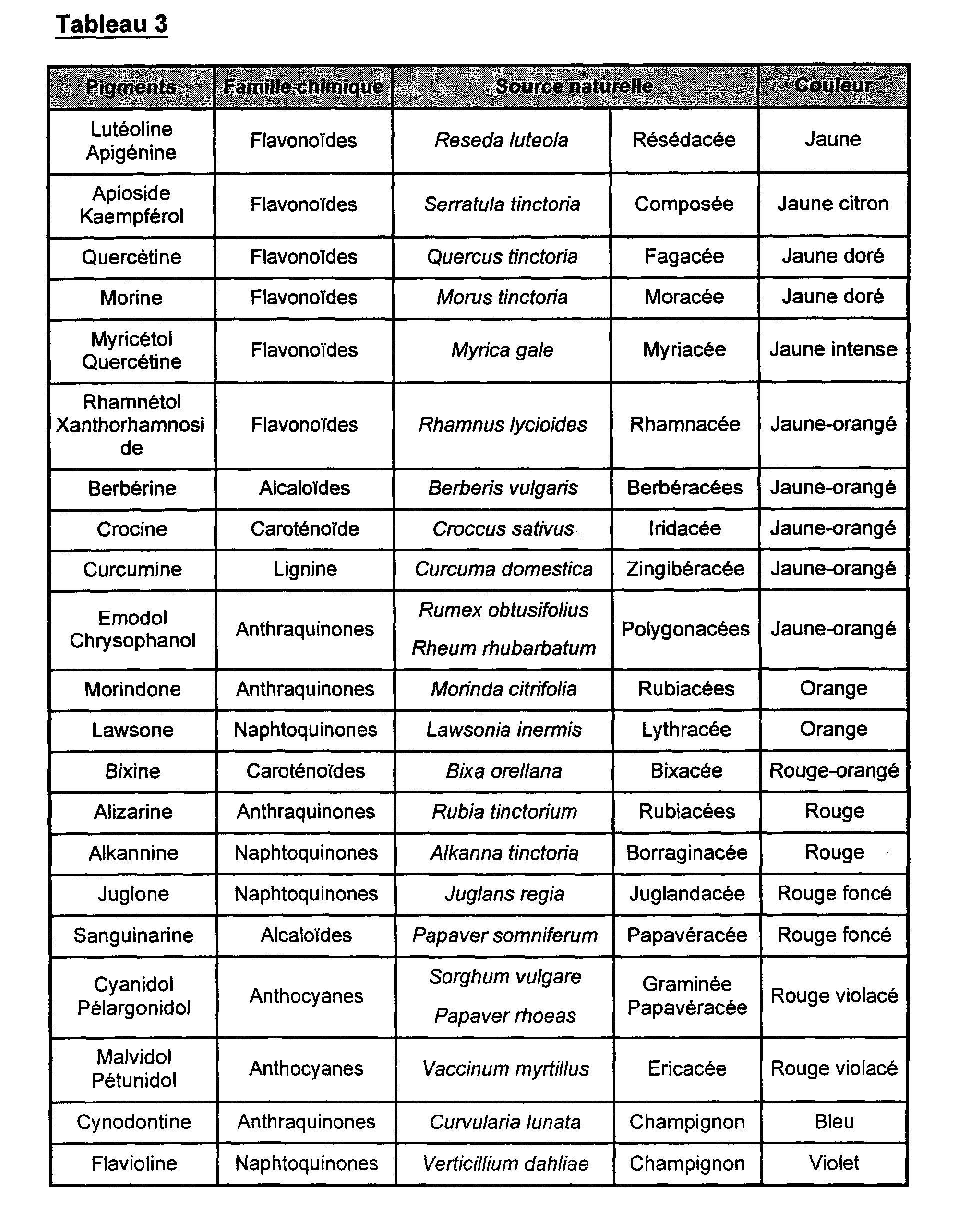

Actuellement, la majorité des matières colorantes industrielles sont produites par synthèse chimique.

Une application de la présente invention est l'obtention de substances à pouvoir colorant à partir de co-cultures impliquant des cellules végétales.

Cette approche requiert de considérer *a priori* les éléments suivants :

En premier lieu, il convient de déterminer si les co-cultures sont réalisables dans les milieux traditionnels de l'un ou l'autre des types cellulaires mis en culture simultanément ou bien si un milieu de culture consensuel doit être trouvé, sachant que les milieux de culture sont très différents pour chacune des entités cellulaires. En effet, les deux domaines que sont la microbiologie et la biotechnologie végétale ont entraîné le développement de milieux de culture spécifiques. Ainsi, de nombreux champignons sont classiquement cultivés sur des milieux de type *potato carrot agar* ou *potato dextrose agar* (PDA), dont le protocole de préparation est indiqué ci-dessous. Ces milieux sont mal contrôlés (milieux complexes), du fait de la variabilité des ingrédients de base. En effet, les pommes de terre et les carottes utilisés pour préparer ces milieux peuvent appartenir à des variétés différentes et avoir été cultivées de multiples façon (sol, climat, amendements...). En revanche, les cellules végétales peuvent être cultivées en utilisant des milieux bien définis, par exemple de type Murashige & Skoog, ou Gamborg, par exemple. Classiquement, les milieux de culture pour cellules végétales sont des milieux parfaitement définis.

La composition des milieux de cultures cités ci-dessus est la suivante :
**potato carrot agar** : Pomme de terre : 20.0 g, Carottes : 20.0 g, Agar : 15.0 g, Eau distillée : 1.0 L

Couper les pommes de terre et les carottes et les faire cuire dans de l'eau durant 1/2 heure. Filtrer au travers d'un linge fin et ajouter l'Agar.

### potato dextrose agar (PDA)

Pommes de terre découpées : 300.0 g, Glucose, 20.0 g, Eau distillée 1.0L.

Faire bouillir les pommes de terre finement coupées dans 500 ml d'eau jusqu'à cuisson totale. filtrer à travers un linge fin et ajouter de l'eau QSP 1.0 L. Chauffer pour dissoudre l'Agar dans le filtrat. Ajouter le glucose avant la stérilisation.

### Exemple de milieu de base Gamborg, (Murashige, Thorpe, Vasil, In Vitro, Vol. 12(7) : 473-478, 1976) :

| Pour 1 litre : | | |
|---|---|---|
| Macro-éléments de Gamborg | 100 | ml |
| Micro-élément de Gamborg | 1 | ml |
| Vitamines de Gamborg | 2 | ml |
| Fer EDTA | 10 | ml |
| Acide 2-4-dichloro phénoxy acétique | 10⁻⁴ (ou 2,4-D10⁻⁴M) | 10 ml |
| Kinétine 10⁻⁴ | 0,6 | ml (0,06mg) |
| Saccharose | 20 | g |
| Eau distillée qsp | 1 | Litre |
| pH avant stérilisation | 5,8 | UpH |
| Stérilisation 115 ou 121°C durant 20 à 40 minutes | | |

Pour obtenir un milieu gélosé on ajoute Agar 8 g.

| *Macro-éléments de Gamborg* | mg/l | mM |
|---|---|---|
| KNO₃ | 2500 | 25.0 |
| CaCl₂, 2H₂O | 150 | 1.0 |
| MgSO₄, 7 H₂O | 250 | 1.0 |
| (NH₄)₂SO₄ | 134 | 1.0 |
| NaH₂PO₄, 1 H₂O | 150 | 1.1 |

| *Micro-éléments de Gamborg* | mg/l | µM |
|---|---|---|
| Kl | 0,75 | 4.5 |
| H₃BO₃ | 3.0 | 50.0 |
| MnSO₄, 1 H₂O | 10.0 | 60.0 |
| ZnSO₄, 7H₂O | 2.0 | 7.0 |
| Na₂MoO₄, 2 H₂O | 0.25 | 1.0 |
| CuSO₄, 5 H₂O | 0.025 | 0.1 |
| CoCl₂, 6 H₂O | 0.025 | 0.1 |

| *Vitamines de Gamborg* | mg/l | µM |
|---|---|---|
| MYO - INOSITOL | 100.0 | 555.5 |
| NICOTINIC ACID | 1.0 | 8.0 |
| PYRIDOXINE HCI (B₆) | 1.0 | 4.8 |

### Exemple de milieu de Base Murashige & Skoog (Détaillé)

| | | |
|---|---|---|
| Macro éléments de Skoog | 100 | ml/l |
| Micro éléments de Skoog | 1 | ml |
| Vitamines de Skoog | 2 | ml |
| Fer EDTA | 10 | ml |
| 2-4 D 10⁻⁴ | 10 | ml |
| Kinétine 10⁻⁴ | 0,6 | ml |
| Saccharose | 30 | g |
| Eau distillée | qsp | 1 Litre |
| pH avant stérilisation | 5,8 | UpH |
| Stérilisation 115 ou 121°C durant 20 à 40 minutes | | |

Pour obtenir un milieu gélosé solide, on ajoute :

| | |
|---|---|
| Agar | 8 g |

| Macro éléments en mg/l | **SKOOG** |
|---|---|
| *KNO*_{*3*} | 1900 |
| *NH*_{*4*}*NO*_{*3*} | 1650 |
| *MgSO*_{*4*}, *7 H*_{*2*}*O* | 370 |
| *CaCl*_{*2*}, 2 *H*_{*2*}*O* | 440 |
| *KH*_{*2*}*PO*_{*4*} | 170 |

| Micro éléments en mg/l | **SKOOG** |
|---|---|
| *CuSO*_{*4*}, *5 H*_{*2*}*O* | 0.025 |
| *MnSO*_{*4*}, *1 H*_{*2*}*O* | 16.9 |
| *Kl* | 0.83 |
| *Na*_{*2*}*MoO*_{*4*}, *2 H*_{*2*}*O* | 0.25 |
| *ZnSO*_{*4*}, *7 H*_{*2*}*O* | 10.6 |
| *H*_{*3*}*BO*_{*3*} | 6.2 |
| *CoCl*_{*2*}, *6 H*_{*2*}*O* | 0.025 |
| Vitamines mg/l | SKOOG |
| *Myoinositol* | 100 |
| *Acide nicotinique* | 0.5 |
| *Pyridoxine* | 0.5 |
| *Thiamine* | 0.1 |
| *FeSO*_{*4*}, *7 H*_{*2*}*O* | 27.8 |
| *Na*_{*2*} *EDTA* | 37.3 |

Outre la question du milieu de co-culture, il faut déterminer s'il est possible d'établir des co-cultures *stables,* dans le sens où un équilibre existe entre les différentes lignées et qu'aucune d'entre elles n'est éliminée au cours des repiquages successifs de la co-culture.

Enfin, la production des métabolites recherchés peut, selon les associations réalisées, être incertaine et doit être vérifiée.

Les résultats surprenants obtenus et présentés dans les exemples détaillés ci-après, permettent de répondre à ces trois questions. Ces exemples montrent en particulier qu'il est possible, contrairement à toute attente, de co-cultiver de façon stable des cellules végétales et des bactéries, dans un milieu classique pour la culture des cellules végétales, et que cette co-culture permet la production de substances qui ne sont pas produites efficacement en culture pure desdites cellules végétales et bactéries (exemple 1). L'exemple 2 montre, là encore contrairement à toute attente, que la co-culture de cellules végétales et de fungi dans un milieu classique de culture de cellules végétales, peut permettre la synthèse de composés absents dans les cultures pures desdits cellules végétales et fungi.

La présente invention porte donc sur des procédés de production de substances d'intérêt, par co-culture de cellules végétales et de phytopathogènes vivants, selon les définitions et dans les conditions décrites ci-dessus pour les co-cultures. Les procédés de l'invention peuvent notamment permettre la production de substances d'intérêt pharmaceutique et/ou cosmétique, en particulier la production de substances à pouvoir colorant.

Dans une réalisation préférée des procédés de l'invention, la co-culture de cellules végétales et de phytopathogènes vivants est stable, ce qui signifie qu'un équilibre s'instaure entre les différentes lignées, qui restent présentes et vivantes au cours des repiquages de la co-culture. Comme indiqué plus haut, cette stabilité est assurée dès lors que tous les types cellulaires de la co-culture sont vivants à l'issue d'un cycle complet du type cellulaire le plus lent.

Dans une mise en oeuvre préférée des procédés de l'invention, la co-culture est réalisée dans un fermenteur ou enceinte étanche stérile et/ou stérilisable, agitée et/ou soumise à agitation. Des exemples de fermenteurs utilisables dans les procédés de l'invention sont les fermenteurs à agitation de marque RUSHTON, AIRLIFT, DRAFT Tube (DRAUGHT Tube aux USA) ou encore de type piston. Dans les procédés de l'invention, les organismes peuvent être cultivés ensemble ou séparés par une membrane en système batch, fed batch ou continu. Des procédés physiques permettant de séparer deux cultures par une membrane sont décrits dans le brevet US.5,665,596.

Dans un système batch, les cellules se multiplient dans le fermenteur jusqu'à épuisement du milieu de culture.

Dans un système fed batch, en fin de culture batch, une partie de la culture est prélevée (de 50 à 80% en général). Le volume prélevé est remplacé par du milieu neuf. Plusieurs cycles identiques sont possibles.

Dans un système continu, en fin de phase exponentielle de culture en mode batch, le fermenteur est récolté en continu et en même temps rempli du même débit en milieu neuf que le débit prélevé.

Ces 3 techniques de production doivent être menées de façon axénique.

Dans une mise en oeuvre préférée des procédés de l'invention, la co-culture est réalisée dans un milieu de culture initialement parfaitement défini. En particulier, le milieu de culture utilisé peut être un milieu entièrement synthétique. Il peut être choisi par exemple parmi les milieux de culture classiquement utilisés pour cultiver des cellules végétales. Bien entendu, la co-culture entraîne la production de substances qui vont par la suite rendre ce milieu plus complexe. Les molécules produites par chacun des types cellulaires en culture pourront donc avoir un effet, direct ou indirect, sur les cellules de l'autre type.

Dans les procédés de l'invention, les cellules végétales peuvent être isolées ou organisées en structures pluricellulaires. En particulier, il peut s'agir de cellules dédifférenciées (exemple 2), ou d'organes, par exemple de racines (exemple1).

Dans la description des procédés de la présente invention, le terme « phytopathogène » ne se limite pas aux organismes naturellement capables d'infecter des plantes (phytopathogènes authentiques), même si les procédés impliquant des phytopathogènes authentiques constituent une classe particulière de procédés selon l'invention. L'invention concerne aussi des procédés de production de substances d'intérêt par co-culture de cellules végétales avec des cellules avec lesquelles elles n'ont jamais été en contact. Dans les procédés de l'invention, des cellules d'origine végétale sont co-cultivées avec des cellules, tissus ou organismes qui peuvent être choisis parmi les archébactéries, les cyanobactéries, et de façon générale, les bactéries, les levures, les champignons, les cellules animales, les insectes ou les virus. Ces cellules, tissus ou organismes sont désignés ici par le terme « phytopathogène ».

Dans une mise en oeuvre particulièrement préférée des procédés de l'invention, la co-culture de cellules végétales et de phytopathogènes permet la synthèse de substances qui ne sont pas produites dans une culture pure de l'un quelconque des éléments co-cultivés, ou qui sont produites à des niveaux faibles. La co-culture permet alors une interaction vivant-vivant entre ces éléments, qui induit avantageusement une modification du métabolisme d'un ou plusieurs des types cellulaires co-cultivés, entraînant une augmentation de la synthèse de substances intéressantes. Lors de la mise en oeuvre des procédés de l'invention, la synthèse d'une substance intéressante peut être augmentée d'un facteur 2 à 3, ou d'un facteur 3 à 10, voire d'un facteur supérieur à 10 et, le cas échéant, d'un facteur supérieur à 100.

Les substances d'intérêt dont la synthèse est favorisée par une co-culture suivant les procédés de l'invention peuvent faire partie du groupe comprenant les phytoalexines, les quinones et leurs dérivés, la lawsone, les polyphénol oxydases, les furocoumarines, les phytochélatines, les peptides et/ou protéines.

Un procédé de l'invention comporte par exemple les étapes suivantes :
A. Ensemencement du milieu de culture avec les cellules végétales et les phytopathogènes choisis,
B. Co-culture des cellules végétales et des phytopathogènes, pendant 1 à 30 jours, en batch et durant toute la production en mode continu,
C. Récupération de la substance recherchée à partir du milieu de culture.

Dans une mise en oeuvre particulière des procédés de l'invention, une substance colorante est produite par co-culture de racines *d'Impatiens balsamina* et de *Streptococcus sp* (exemple 1). Dans une autre mise en oeuvre particulière des procédés de l'invention, des cellules de *Ruta graveolens* sont cultivées en présence de *Verticillium dahliae*, ce qui conduit à une augmentation de la production de flavioline par *Verticillium dahliae*, et une augmentation de la production de furocoumarines par *Ruta graveolens.*

Bien entendu, les substances d'intérêt obtenues par l'un quelconque des procédés de l'invention, font aussi partie de la présente invention. Ceci est en particulier vrai pour les phytoalexines et les substances colorantes obtenues par ces procédés.

Les exemples et figures ci-après, illustrent la mise en oeuvre et l'intérêt de la présente invention, sans toutefois en limiter la portée.

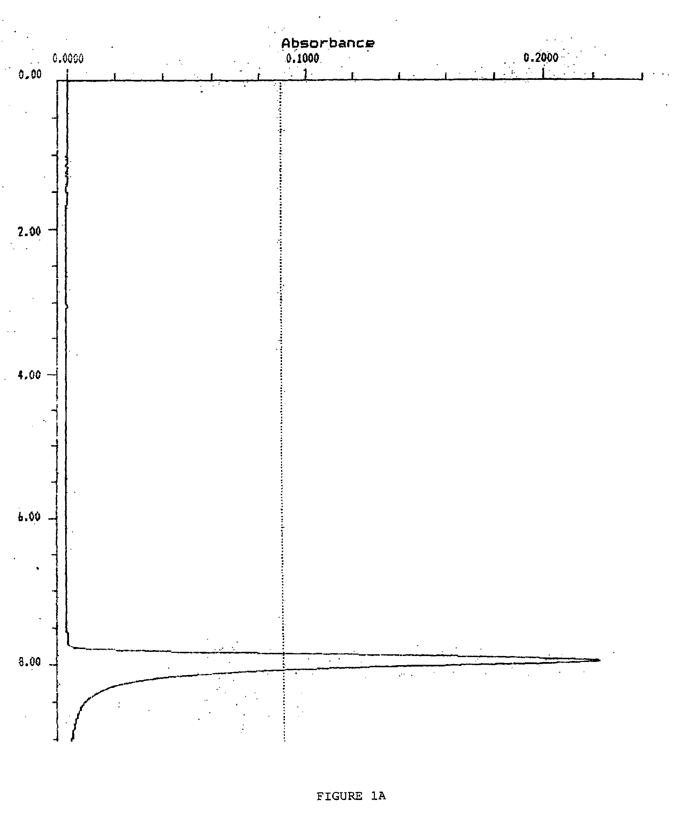

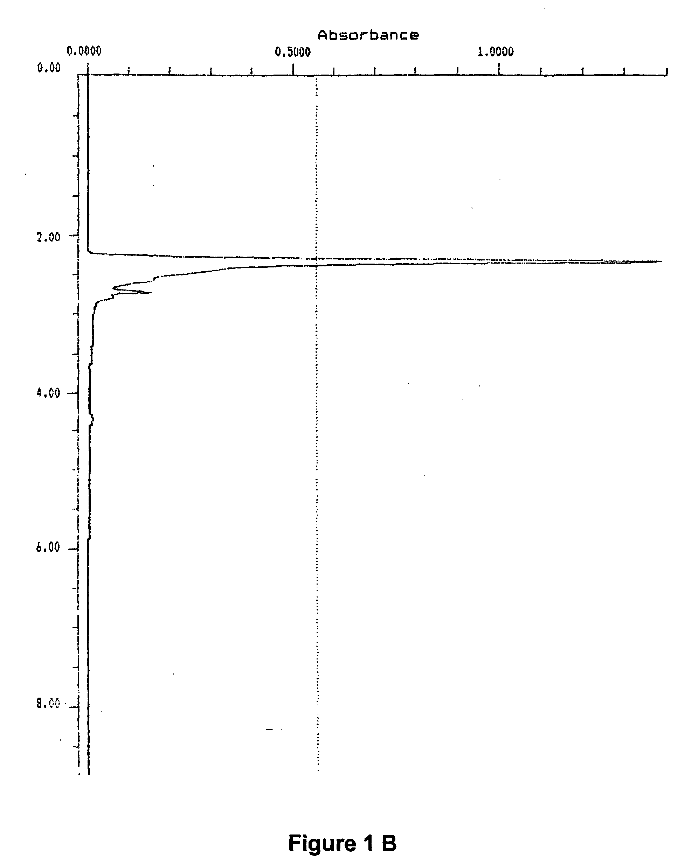

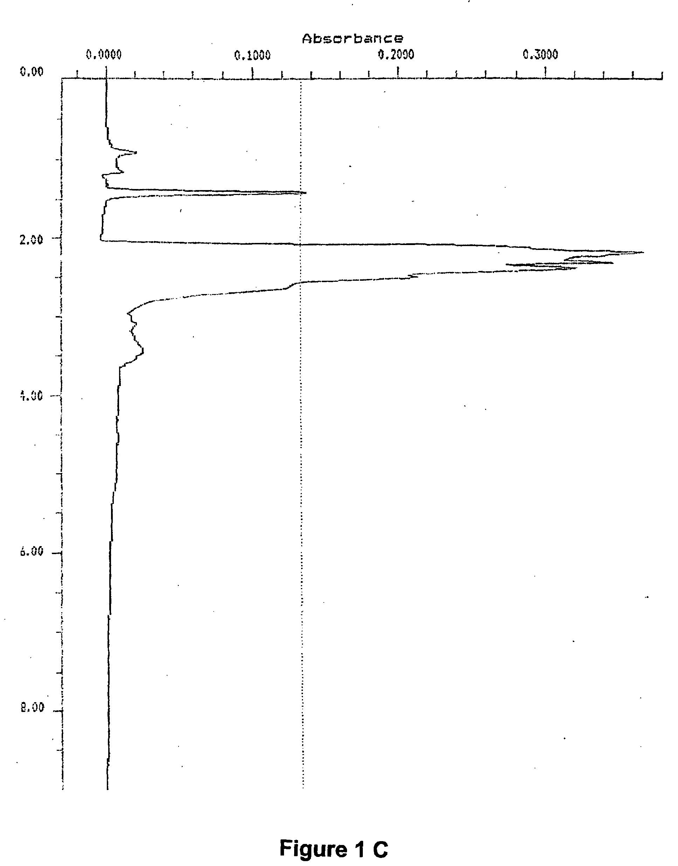

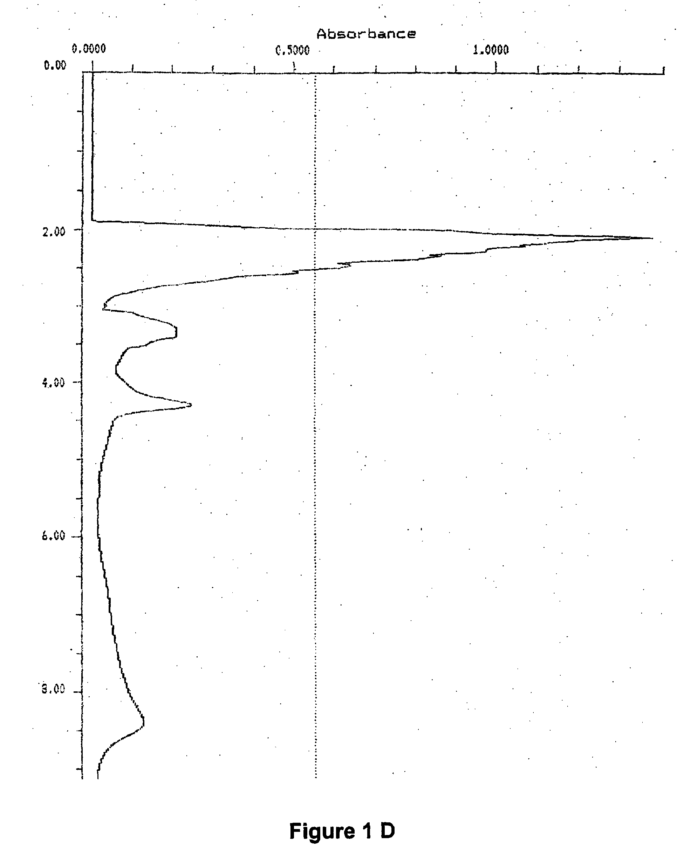

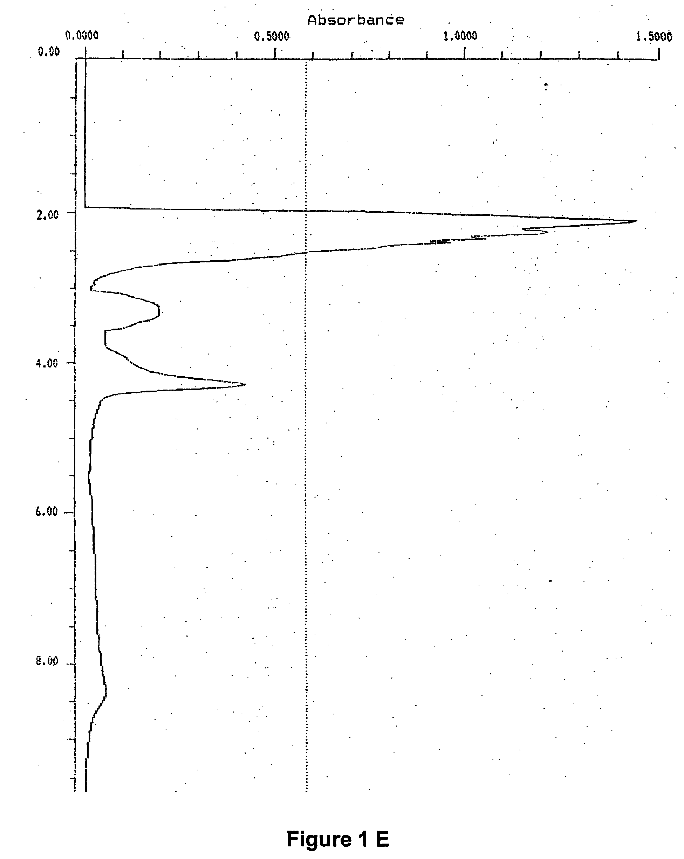

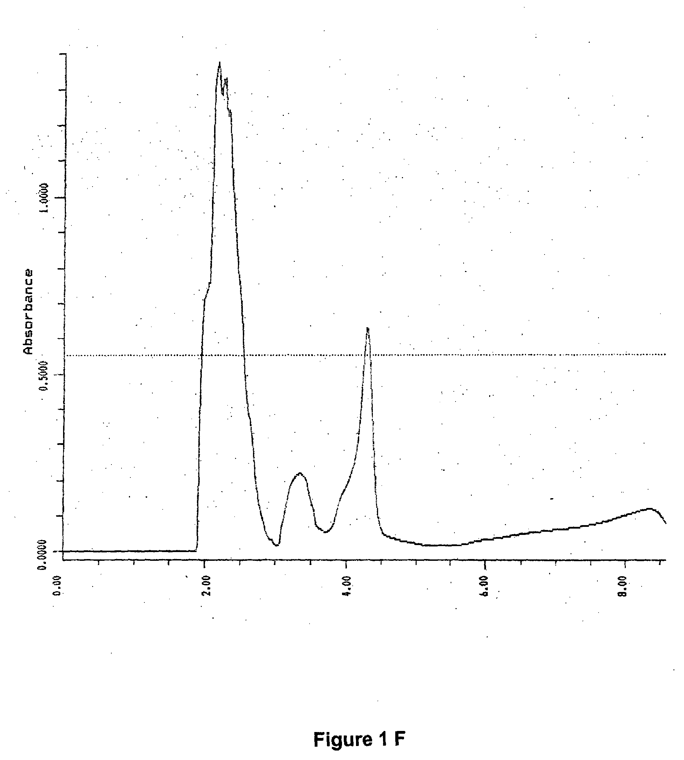

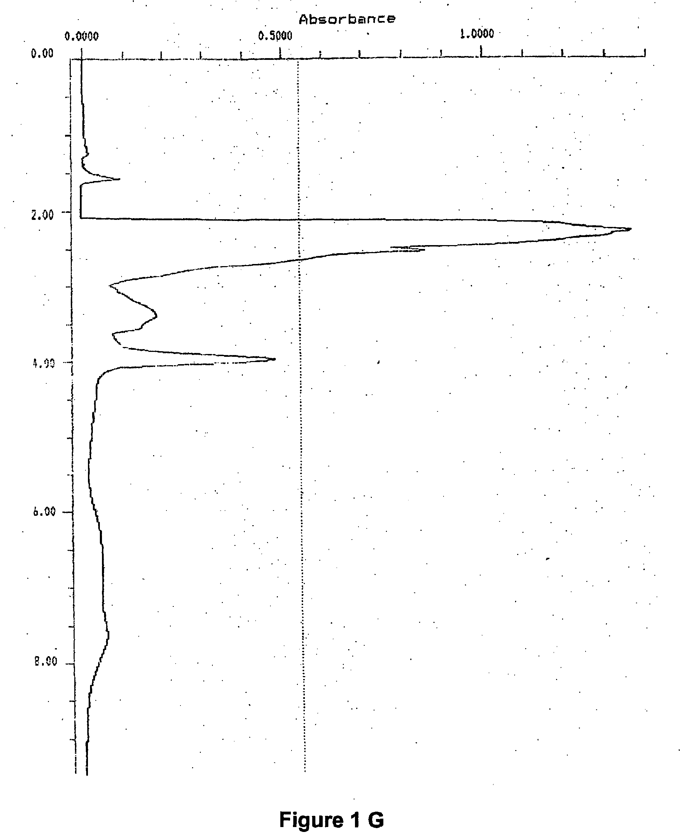

Les chromatogrammes présentés dans le figure 1 (extraction éthanolique) montrent l'influence des différentes conditions de culture sur la composition des racines d'*Impatiens balsamina.*
Figure 1A : Chromatogramme d'une solution de lawsone pure
Figure 1B : Chromatogramme du milieu de culture vierge
Figure 1C: Chromatogramme de racines d'*Impatiens balsamina* autoclavées, mises en présence d'un *Streptocoque* vivant
Figure 1D : Chromatogramme de racines d'*Impatiens balsamina* élicitées par le *Streptocoque* autoclavé
Figure 1E : Chromatogramme de racines d'*Impatiens balsamina* saines, en culture seule
Figure 1F : Chromatogramme d'une co-culture de racines d'*Impatiens balsamina* contaminées par le *Streptocoque* vivant
Figure 1 G: Chromatogramme de racines d'*Impatiens balsamina* recontaminées par le *Streptocoque* vivant

### EXEMPLES

### Exemple 1 : Co-cultures in vitro de racines d'Impatiens balsamina et de Streptococcus sp. :

*Impatiens balsamina* appartient à la famille des Balsaminacées. Cette plante produit entre autres une substance colorante retrouvée dans le Henné : la lawsone ou 2 hydroxy 1,4 naphytoquinone. Des souches racinaires obtenues en laboratoire produisent également de la lawsone en faible quantité.

De façon surprenante, les inventeurs ont observé deux comportements différents de la même souche racinaire d'*Impatiens balsamina* cultivées de la même manière en Erlemeyer de 250 ml sur shaker rotatif repiquées tous les 14 jours sur le même milieu de base qui est le suivant :

| | | |
|---|---|---|
| CaCl₂, 2H₂O | 0.44 | g/L |
| KH₂PO₄ | 0.17 | g/L |
| MgSO₄, 7H₂O | 0.37 | g/L |
| NH₄NO₃ | 1.65 | g/L |
| CuSO₄, 5H₂O | 0.025 | mg/L |
| CoCl₂, 6H₂O | 0.025 | mg/L |
| Na₂MoO₄, 2H₂O | 0.25 | mg/L |
| Kl | 0.83 | mg/L |
| ZnSO₄, 7H₂O | 8.6 | mg/L |
| H₃BO₃ | 6.2 | mg/L |
| MnSO₄, 4H₂O | 22.3 | mg/L |
| Myoinositol | 100 | mg/L |
| Acide Nicotinique | 0.5 | mg/L |
| Pyridoxine, HCI (4°C) | 0.5 | mg/L |
| Thiamine, HCI (4°C) | 0.1 | mg/L |
| Glycine | 2 | mg/L |
| FeSO₄, 7H₂O | 27.8 | mg/L |
| Sequestrène 330 Fe | 37.3 | mg/L |
| Acide Naphtalène Acétique | 1 | mg/L |
| Kinétine | 0.06 | mg/L |
| Saccharose | 30 | g/L |
| Eau distillée | qsp 1 | Litre |
| pH avant stérilisation | 5.8 | UpH |
| Stérilisation suivant volumes : de 15' à 40' à 115°C ou 121°C | | |

Les racines sont inoculées à raison de 5 g de matière fraîche pour 100 ml de culture.

Une des deux cultures présente des racines beiges alors que la seconde est rouge oranger. Les deux cultures sont stables depuis plusieurs années.

La culture rouge a été filtrée et son milieu a été analysé. Ce dernier contient des bactéries. Un isolement a pu révéler qu'il s'agit d'une culture bactérienne pure qui a été identifié par l'Institut Pasteur comme étant un *Streptococcus sp.*

Cette dernière a été cultivée à l'obscurité à 26,5°C sur milieu LPG, plus propice à son développement, de composition suivante :

| | | |
|---|---|---|
| Extrait de levure | 5 | g/L |
| Glucose | 10 | g/L |
| Peptone | 5 | g/L |
| Agar | 15 | g/L Culture sur gélose |
| Eau distillée | qsp 1 | Litre |
| Stérilisation suivant volumes : de 15' à 40' à 115°C ou 121°C. | | |

Pour vérifier que la présence de la coloration rouge de la souche d'*Impatiens balsamina* est effectivement induite par la bactérie, des racines beiges (non contaminées) ont été infectées par le Streptocoque cultivé sur milieu LPG. Dans tous les cas d'infection, l'apparition de la couleur rouge est observée.

Le phénomène est stable au fil des repiquages, et une co-culture vraie et stable s'installe, conservant aux racines d'*Impatiens balsamina* leur coloration rouge.

Ces mêmes bactéries, tuées par la chaleur puis ajoutées à des racines non pigmentées, ne produisent aucun changement. Le tableau ci-après reprend les différents essais réalisés :

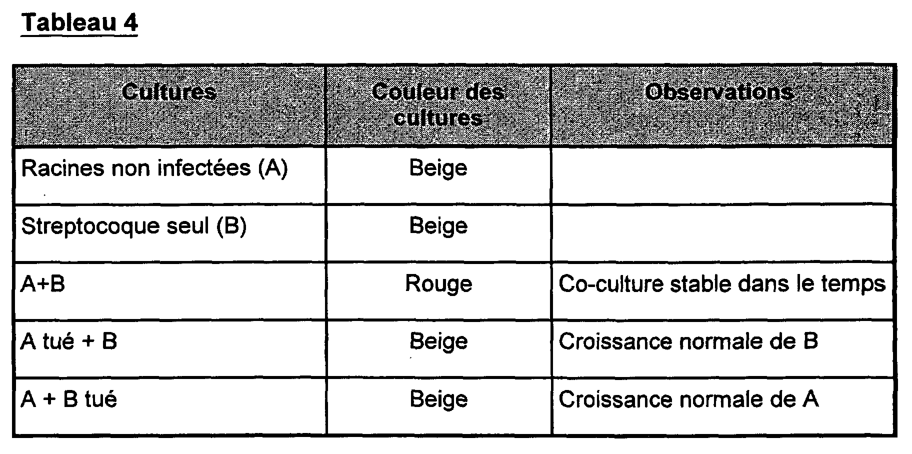

Le phénomène n'est donc pas dû à une élicitation traditionnelle mais bien à une co-culture vraie de cellules vivantes.

Le premier effet surprenant observé ici est l'adaptation des Streptocoques au milieu de culture des cellules végétales.

Le deuxième effet surprenant est la stabilité des co-cultures dans le temps : aucune des deux lignées cellulaires ne prend le pas sur l'autre.

Différents dosages en Lawsone (jaune/oranger) montrent que celle-ci peut être élicitée par le streptocoque tué, mais ceci ne peut expliquer l'apparition de la coloration rouge vif des racines obtenues en co-culture.

Les chromatogrammes présentés dans le figure 1 (extraction éthanolique) montrent l'influence des différentes conditions de culture sur la composition des racines d'*Impatiens balsamina*.

### Exemple 2 : Co-cultures in vitro de cellules de Ruta graveolens (la Rhue) et de Verticillium dahliae

Cet exemple illustre la possibilité de cultiver une cellule dédifférenciée d'origine végétale en présence d'un champignon phytopathogène.

*Ruta graveolens* est une plante de la famille des Rutacées qui synthétise des furocoumarines dont le psoralène et certains de ses dérivés méthoxylés : le 5-MOP (bergaptène), 8-MOP (xanthotoxine) et 5,8-MOP (isopimpinelline). Ces furocoumarines sont des métabolites secondaires produits en réponse à une agression par un phytopathogène. Ce sont donc des phytoalexines limitant la prolifération des phytopathogènes.

*Verticillium dahliae* est un champignon inférieur phytopathogène appartenant à la famille des Adélomycètes de l'ordre des Hyphales parasitant un grand nombre d'espèces végétales dont le dahlia, le coton, la pomme de terre, le cacao, la tomate, l'aubergine ou encore le fraisier. Il synthétise entre autres une naphtoquinone : la flavioline ou 2,5,7 trihydroxy 1,4 naphtoquinone.

Sa culture s'effectue sur milieu PDA de composition :

| | | |
|---|---|---|
| Broyats de pomme de terre | 200 | g/L |
| Glucose | 20 | g/L |
| (Agar | 15 | g/L) |
| pH avant stérilisation | 5,6 | UpH |

Les cultures s'effectuent à l'obscurité en boite de Pétri à 26,5°C.
Son passage en milieu liquide s'effectue en milieu pour croissance végétale B5 D2 à l'obscurité :

Macro-, micro-éléments, vitamines et fer du milieu de Gamborg,

| | | |
|---|---|---|
| Acide 2,4 dichlorophenoxyacétique 10⁻⁴ M | 2 | mg/L |
| Saccharose | 30 | g/L |
| PH avant stérilisation | 5,8 | UpH |

*Ruta graveolens* est cultivée également sur milieu B5 D2 à la lumière.

On effectue les cultures pures de chacune des entités cellulaires, et des cultures élicitées par les cellules de l'autre type tuées par la chaleur et enfin la co-culture vraie entre *Ruta graveolens* et *Verticillium dahliae.*

Chaque entité cellulaire est extraite puis analysée. Les résultats sont les suivants :

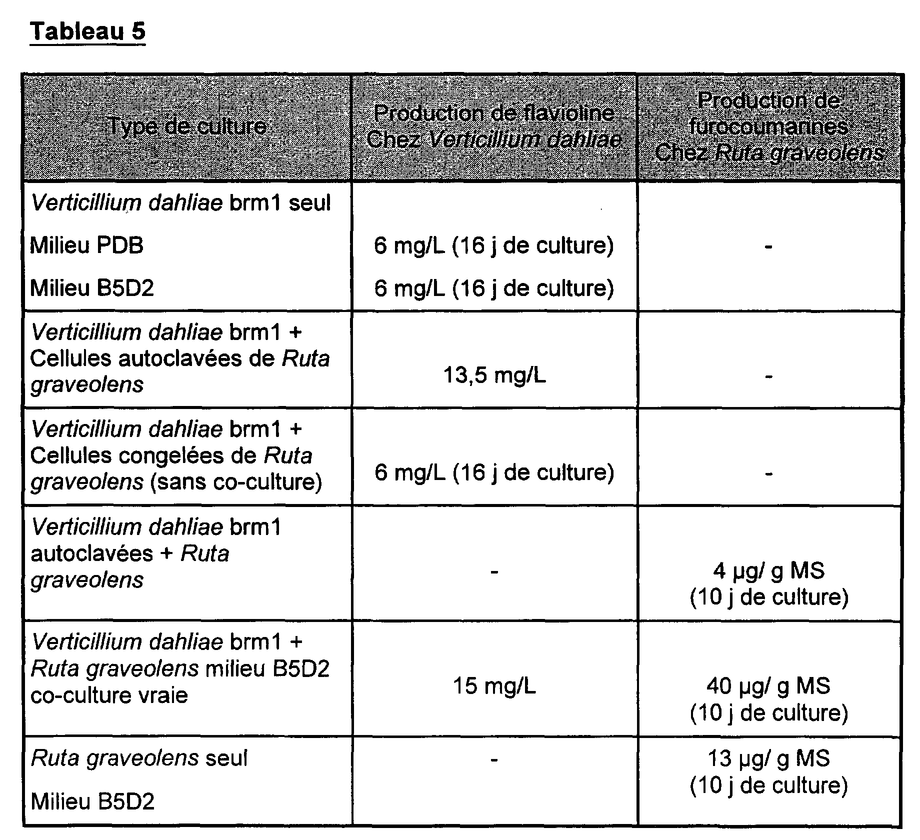

Flavioline : extracellulaire
Furocoumarines : intracellulaires
MS : Matière sèche en cellule végétale

Les essais ont été réalisés en culture éclairée plus adaptée à la croissance de la souche de *R. graveolens*

Ces expériences montrent que la production de flavioline est optimisée dans des conditions de co-culture vraie. De plus, les cellules congelées (non viables) donnent les mêmes résultats que la culture pure de champignon. Ce résultat montre clairement que l'interaction vivant/vivant est nécessaire pour augmenter la synthèse d'un facteur 2,5. L'autoclavage montre une dénaturation des composants de la cellule végétale. Ces derniers se comportent comme des éliciteurs moins efficaces que la co-culture vraie.

En ce qui concerne la production de furocoumarines, ces résultats montrent que l'ajout de champignon tué à la culture de *Ruta graveolens* a un effet inverse à celui recherché, puisqu'on ne produit que le tiers des furocoumarines produites en culture pure. La co-culture permet de produire 3 fois plus de furocoumarines que la culture pure.

Ces résultats montrent donc clairement les effets de l'interaction vivant/vivant, qui permet une augmentation de la biosynthèse de composés dans les deux lignées cellulaires. Ces composés sont de natures très différentes : colorants et phytoalexines, montrant ainsi la richesse de ce type de technique.

## Revendications

1. Co-culture stable *in vitro* de cellules d'origine végétale et de phytopathogènes, permettant de produire des substances d'intérêt.

2. Co-culture selon la revendication 1, **caractérisée en ce que** les cellules d'origine végétale sont individualisées ou organisées en structures pluricellulaires.

3. Co-culture selon la revendication 1 ou 2, **caractérisée en ce que** les cellules végétales sont dédifférenciées.

4. Co-culture selon les revendications 1 à 3, **caractérisée en ce que** les phytopathogènes sont des archébactéries, des bactéries, des protistes, des fungi, des cellules animales, des insectes, des virus ou des levures.

5. Co-culture selon les revendications 1 à 4, dans laquelle les phytopathogènes sont des cellules procaryotes.

6. Co-culture selon les revendications 1 à 4, dans laquelle les phytopathogènes sont des fungi.

7. Co-culture selon les revendications 1 à 4, dans laquelle les phytopathogènes sont des virus.

8. Co-culture selon l'une quelconque des revendications 1 à 7, **caractérisée en ce que** les phytopathogènes sont des phytopathogènes authentiques.

9. Co-culture selon les revendications 1 à 4, dans laquelle les phytopathogènes sont des levures.

10. Co-culture selon les revendications 1 à 5, dans laquelle les cellules végétales sont des racines d'*Impatiens balsamina* et le phytopathogène est *Streptococcus sp.*

11. Co-culture selon les revendications 1 à 4 et 6, dans laquelle les cellules végétales sont des cellules de *Ruta graveolens* dédifférenciées et le phytopathogène est *Verticillium dahliae.*

12. Co-culture selon l'une quelconque des revendications 1 à 11, **caractérisée en ce que** le milieu de culture est initialement parfaitement défini.

13. Co-culture selon l'une quelconque des revendications 1 à 12, permettant la production de substances d'intérêt pour l'alimentation, l'agrochimie, la pharmacie ou la cosmétique.

14. Co-culture selon l'une quelconque des revendications 1 à 13, **caractérisée en ce qu'**elle produit une substance d'intérêt de façon plus efficace que les cultures pures desdites cellules végétales ou desdits phytopathogènes de ladite co-culture, le facteur d'augmentation de la synthèse de ladite substance d'intérêt par ladite co-culture, par rapport à la culture pure la plus efficace, étant compris entre 2 et 3, ou entre 3 et 10, ou supérieur à 10, ou supérieur à 100.

15. Procédé de production en fermenteur, de substances d'intérêt, par co-culture de cellules végétales et de phytopathogènes vivants.

16. Utilisation d'un procédé selon la revendication15, pour la production de substances d'intérêt pour l'alimentation, l'agrochimie, la pharmacie et/ou la cosmétique.

17. Utilisation d'un procédé selon la revendication15, pour la production de substance à pouvoir colorant.

18. Procédé ou utilisation selon les revendications 15 à 17, dans lequel la co-culture est stable.

19. Procédé ou utilisation selon les revendications 15 à 18, dans lequel la co-culture est réalisée dans un fermenteur ou enceinte étanche stérile et/ou stérilisable, agitée et/ou soumise à agitation.

20. Procédé ou utilisation selon les revendications 15 à 19, dans lequel les organismes sont cultivés ensemble ou séparés par une membrane en système batch, fed batch ou continu.

21. Procédé ou utilisation selon les revendications 15 à 20, dans lequel le milieu de culture est initialement parfaitement défini.

22. Procédé ou utilisation selon les revendications 15 à 21, dans lequel les cellules végétales sont isolées ou organisées en structures pluricellulaires.

23. Procédé ou utilisation selon les revendications 15 à 21, dans lequel les cellules végétales sont dédifférenciées.

24. Procédé ou utilisation selon les revendications 15 à 23, dans lequel les phytopathogènes sont choisis parmi les archébactéries, les bactéries, les protistes, les fungi, les cellules animales, les insectes ou les virus.

25. Procédé ou utilisation selon les revendications 15 à 24, dans lequel les phytopathogènes sont des phytopathogènes authentiques.

26. Procédé ou utilisation selon les revendications 15 à 25, comprenant les étapes suivantes :
A. Ensemencement du milieu de culture avec les cellules végétales et les phytopathogènes choisis,
B. Co-culture des cellules végétales et des phytopathogènes, pendant 1 à 30 jours en batch, et durant toute la production en mode continu,
C. Récupération de la substance recherchée à partir du milieu de culture.

27. Procédé ou utilisation selon les revendications 15 à 26, pour la production d'une substance colorante par co-culture de racines d'Impatiens balsamina et de Streptococcus sp.

28. Procédé selon les revendications 15 ou 18 à 27, dans lequel la co-culture de cellules végétales et de phytopathogènes permet de produire une substance d'intérêt de façon plus efficace que les cultures pures desdites cellules végétales ou desdits phytopathogènes de ladite co-culture, le facteur d'augmentation de la synthèse de ladite substance d'intérêt par ladite co-culture, par rapport à la culture pure la plus efficace, étant compris entre 2 et 3, ou entre 3 et 10, ou supérieur à 10, ou supérieur à 100.

29. Utilisation d'un procédé selon les revendications 15 ou 18 à 28, dans lequel la co-culture de cellules végétales et de phytopathogènes permet la synthèse de substances qui ne sont pas produites efficacement dans une culture pure.

30. Utilisation selon la revendication 28, **caractérisée en ce que** lesdites substances font partie du groupe comprenant les phytoalexines, les quinones et leurs dérivés, la lawsone, les polyphénol oxydases, les furocoumarines, les phytochélatines, les peptides ou les protéines

31. Phytoalexines et substances colorantes obtenues par l'un quelconque des procédés 15 ou 18 à 27.
